# EUROPEAN PATENT SPECIFICATION

(11) **EP 1 325 024 B1**
(45) Date of publication and mention of the grant of the patent: **20.04.2011**
(21) Application number: 01965483.9
(22) Date of filing: 17.09.2001
(51) Int. Cl.: C12Q 1/04

(54) **SELECTIVE GROWTH MEDIA**
SELEKTIVE WACHSTUMSMEDIEN
MILIEU DE CROISSANCE SELECTIF

(30) Priority: 18.09.2000 EP 00308139
(43) Date of publication of application: 09.07.2003
(73) Proprietor: The Newcastle upon Tyne Hospitals National Health Service Trust, Newcastle-Upon-Tyne, NE7 7DN (GB)
(72) Inventor: PERRY, John, David Microbiology Dept., Newcastle-Upon-Tyne Tyne & Wear NE7 7DN (GB)
(74) Representative: Jones, Helen M.M.
(86) International application number: PCT/GB2001/004144
(87) International publication number: WO 2002/024725

(56) References cited:
- WO-A-02/22785
- WO-A-96/30543
- US-A- 4 016 148
- US-A- 5 573 947
- M.M.GIBSON ET AL: "Genetic Characterization and Molecular Cloning of the Tripeptide Permease (tpp) Genes of Salmonella typhimurium" J.BACTERIOL., vol. 160, no. 1, 1984, pages 122-130, XP000973938
- MILLER R.G. ET AL.: "Xylose-Lysine-Tergitol 4: An Improved Selective Agar Medium for the Isolation of Salmonella" POULTRY SCIENCE, vol. 70, 1991, pages 2429-32, XP000973966
- PARK C. E. ET AL.: "selective enrichment of shigella in the presence of E. coli by use of 4-Cl-2-cyclopentylphenyl beta-D-galactopyranoside" CAN. J. MICROBIOL., vol. 22, - 1976 pages 654-657, XP001038648
- JOHNSTON M. A. ET AL.: "Selective inhibition of E. coli in the presence of S. typhimurium by phenethy beta-D-galactopyranosidel" APPL. MICROBIOL., vol. 15, - 1967 pages 1223-1228,
- ALLEN ET AL.: "Phosphonopeptides as antibacterial agents: alaphosphin and related phosphonopeptides" ANTIMICROBIAL AGENTS AND CHEMOTHERAPY, vol. 15, no. 5, 1979, pages 684-695,

## Description

This invention relates to media which support the growth of some microbes but are designed to inhibit the growth of other types of microbes for culturing microbes in a sample containing a mixture of species. Such media are termed selective media. This invention comprises a novel type of selective media. The preferred embodiment provides a selective media for the detection of target Salmonella species, which allows Salmonella species to survive and grow whilst inhibiting the growth of many other commensal bacteria.

Diagnostic microbiology laboratories rely heavily on culture media for the growth of bacterial pathogens in order to diagnose disease or detect bacteria in commercial products such as food-stuffs. These media generally contain non-specific blends of proteins ('peptones') to support bacterial growth and may employ agar as a solidifying agent. A vast array of media is available and these may range from general purpose media for the growth of as many species as possible to media designed for the specific detection of target pathogens. Media for specific pathogens are generally termed 'selective' agars in that they generally attempt to inhibit the growth of unrelated bacteria. A wide range of selective ingredients have been employed in media including antibiotics and various chemicals such as sodium chloride and bile salts.

Most media are traditionally non-specific and allow the growth of a wide range of bacteria. Much laborious work is then necessary to identify the potential pathogens from a large amount of bacterial flora. In recent years there has been a shift towards the use of chromogenic media containing enzyme substrates. A wide range of such media is available and different media may be used to specifically target different bacterial pathogens. The key feature of chromogenic media is that the target pathogen cleaves an enzyme substrate present in the agar to produce a coloured product which remains localised within the bacterial colony. This allows differentiation of pathogens, which appear a certain colour, from commensals which are either colourless or appear another colour. Such media maybe quite sophisticated and may include a cocktail of enzyme substrates and other co-factors.

Chromogenic media have the advantage that they are highly specific for the detection of certain pathogens. Because these pathogens generate a certain colour they can be easily differentiated from commensal bacteria thus precluding the need for laborious testing of suspect colonies. Despite their specificity and ease of use many chromogenic media still allow the growth of a wide range of commensal bacteria. It is therefore still necessary to recover suspect bacterial colonies from a morass of other flora. A major challenge therefore in diagnostic microbiology is to design culture media which employ a specific detection method combined with true selectivity against commensal flora.

Various types of selective media are known to those skilled in the art. Some have been designed for the detection of Salmonella including Rambach agar, SM-ID agar and ABC medium. In each case it is of intrinsic importance to the operation of these three media that commensal bacteria hydrolyse a β-galactosidase substrate to generate a strongly coloured product. By comparison, Salmonella colonies on each of these three media produce colonies which are of lighter colour due to a secondary biochemical reaction. A disadvantage of chromogenic selective media is the distinct possibility that suspect colonies may become 'buried' or overgrown by colonies of commensal bacteria if the number of pathogenic bacteria (target species to be detected) in a sample is relatively low compared to the numbers of other bacteria. This problem is exacerbated in examples such as the above mentioned media where the commensal bacteria produce colonies which are darkly coloured and target pathogens produce colonies which are lightly coloured.

There is evidence to suggest that an approach based on the above mentioned chromogenic system is not ideal for optimal recovery of Salmonella. For example, Dusch H. & Altwegg M (J Clin Microbiol 1993 Feb; 31(2):410-2) performed a comparison of Rambach agar, SM-ID medium and traditional agar (Hektoen Enteric) for primary isolation of non-typhi Salmonellae from stool samples. They concluded that, although the two chromogenic media were highly specific, on the basis of the poor sensitivities, Rambach and SM-ID agars could not be recommended as primary plating media when screening for non-typhi Salmonellae.

The inventors performed a study to further examine the selectivity of some different Salmonella agars i.e. their ability to inhibit common commensal bacterial found in stool samples. Six agars were purchased from their respective suppliers and prepared in exact accordance with the manufacturer's instructions. These included Rambach agar (RAM), SM-ID agar, chromagar Salmonella (CS), xylose lysine decarboxylase (XLD) agar, Hektoen enteric agar (HEK) and ABC medium. A total of 433 Gram negative aerobic bacterial strains were inoculated onto each of these agars at an inoculum of 10⁵ colony forming units per ml. This collection comprised 61 strains of Salmonella and 372 strains of non-Salmonella. Whilst all of the media were efficient at supporting growth of Salmonella, most were highly inefficient at inhibiting the growth of non-Salmonellae. This was exemplified by examining the ability of the different media to inhibit the growth of *E*. *coli* the commonest commensal species present in faeces. Of 108 strains tested, over 95% of strains grew on the four chromogenic agars tested: SM-ID, Rambach, ABC medium and Chromagar Salmonella.

It is clear from this example that even among modern, sophisticated chromogenic media there is little selectivity against frequently encountered commensal bacterial. Such media are often claimed to have a high sensitivity and high specificity. They are sensitive in that they will support the growth of a wide range of Salmonella strains and they may be specific in that very few non Salmonella strains produce coloured colonies which are characteristic of Salmonella. However, in terms of the range of species that grow on these media they are far from specific and as already mentioned this is likely to be problematic if a small number of Salmonella bacteria need to be recovered from a large amount of commensal flora.

A selective culture medium containing a β-galactosidase derivative (CPPG) for isolating Shigella or Salmonella species from samples containing E. coli has been described by Park et al in Can. J. Microbiol. 1976, 22, 654-657. The derivative is cleaved by *E*. *coli* β-galactosidase to form a cytotoxic compound but Shigella species did not have such enzyme activity and selectively grew.

In WO96/30543 a culture medium for detection of Salmonella comprises a chromogenic substrate for β-galactosidase-positive species of *Enterobacteriacae* (such as *E*. *coli*) and a mixture of sugars which are metabolised by *Salmonellae* to form acids and a pH indicator to detect acid formation. Bile salts are included to selectively enrich gram-positive (such as Salmonella) species.

Miller et al in PoultryScience 1991, 70, 2429-32, describe the incorporation of tergitol for selective enrichment of *Salmonella spp* as an improvement for the biocide novobiocin in media where Salmonella is detected by use of H₂S indicators, sodium thiosulphate and ferric ammonium citrate.

Antibiotic agents have long been available to those skilled in the art and a wide variety are utilised. One group of antibiotic agents are based on α-amino phosphonic acids. These compounds are α-amino phosphonic acids. Aminoacyl and peptidyl derivatives of 1-aminoalkyl phosphonic acids, for instance having the general formula I 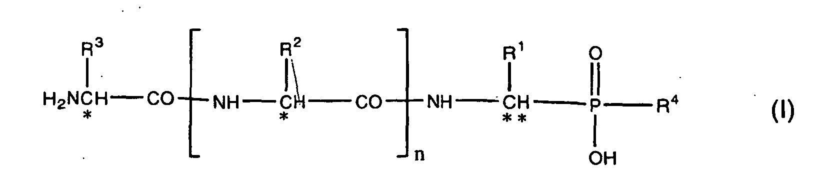 wherein R¹ is hydrogen, lower alkyl, lower cycloalkyl, (lower cycloalkyl)-(lower alkyl), aryl or aryl-(lower alkyl) said substituents other than hydrogen being optionally substituted by one or more of amino, hydroxy, thio, methylthio, carboxy or guanidino so as to form the characterising group of a naturally occurring L alpha-amino acid; R² and R³ each represent the characterising group of an alpha-amino acid of the type normally found in proteins with the proviso that R³ cannot be hydrogen when n is zero and R¹ is hydrogen or phenyl; R⁴ is hydroxy or methyl; n is zero, 1, 2 or 3; the single asterisks denote that the configuration at the carbon atom so-marked is L; and the double asterisk denotes that, when R¹ is other than hydrogen, the configuration at the carbon atom so-marked is that which would be obtained by replacing the carboxyl group of a naturally occurring L alpha-amino acid by a phosphorus moiety [hereinafter referred to as the (R)-configuration], and their pharmaceutically acceptable salts.

The most studied compounds are based on the compound fosfalin (1-aminoethyl phosphonic acid). These compounds are described in US 4,016,148, US 4,331,591 and Atherton F.R et al (1985) Am. Chem. Soc 29, 29-40. Atherton *et al* explain the basis for the antibiotic activity of N-aminoacyl and N-peptidyl α-amino phosphonic acid compounds as being dependent upon the transport into the bacterial cell and the-subsequent ability of intracellular enzymes hydrolyse the compound to release the inhibitory α-amino alkyl phosphonic acid compound. Comparative minimum inhibitory concentrations (MIC's) are given for various bacterial species, including *E. coli.*

In J. Bacteriol. 1984, 160(1), 122-130 Gibson *et al* investigate mutants of *Salmonella typhimurium* having a defective tripeptide permease system by using alafosfalin. Mutants with the defective system are resistant to alafosfalin and this is assumed to be because the compound cannot be transported across the cell membrane. The authors indicate that alafosfalin is inactive against many strains of bacteria because of the high frequency of resistant strains.

Due to the problems encountered with the currently available selective media, this invention provides a unique approach to the design of culture media and the isolation of target bacteria. This involves the use of enzyme substrates which, when utilised by commensal bacteria, cause growth inhibition of the bacteria. This approach has never before been used in the field of diagnostic bacteriology and is substantially different to traditional approaches to selectivity. In order to be inhibited bacteria must be amenable to (i) uptake of the enzyme substrate into the cell, (ii) chemical alteration of the substrate by an enzyme or other metabolic process thus forming an antibacterial agent and (iii) inhibition of a metabolic process by the antibacterial agent. Conversely, target bacteria may survive the presence of the substrate because they (i) do not take up the substrate at a sufficient rate, (ii) are unable to metabolise the substrate or (iii) are intrinsically resistant to any products of substrate metabolism.

This approach allows for the design of selective antibacterial agents on the basis of the metabolic properties of target and commensal bacteria. For example, it is proposed that bacteria may be selectively inhibited on the basis of their possession of a single enzyme which is able to generate a toxic product. This approach is unique to the design of bacterial culture media. Whilst enzyme substrates are widely used in culture media to reveal chromogenic reactions for purposes of differentiation, there is no evidence that enzyme substrates have ever been used for the selective inhibition of bacterial strains in culture media.

According to the invention there is provided a method of culturing bacteria and detecting a target species of bacteria as defined in claim 1.

Preferably the growth medium is a selective medium, that is, it contains other components which promote growth and/or division of target Salmonella species and/or inhibit growth and/or division of commensal species.

The sample may be a direct biological sample, for instance of faeces, urine or blood. Alternatively the direct biological sample may be subjected to one or more cycles of incubation in selective growth media to form the sample used in the method of the invention.

This invention also provides a composition for making up into a bacterial growth medium as defined in claim 12.

Preferably the composition is dry and is suitable for dilution to form the growth medium, for instance solid (gel-type) medium, with water. Preferably the medium is a selective medium for screening bacteria and the composition of the invention from which such a medium may be generated comprises selective compounds which allow target bacteria to grow whilst inhibiting the growth of other commensal bacteria. The presence of the pro-inhibitor results in inhibition of growth after uptake by virtue of the fact that the pro-inhibitor is chemically altered by an enzyme or other metabolic process, thereby releasing the antibacterial toxin. The inhibitor effects its toxicity by inhibition of a metabolic process. We have called the pro-inhibitor a "suicide substrate". The target bacteria (Salmonella) should substantially survive in the presence of the suicide substrate. Survival may be due to the inability of the target species to transport the pro-inhibitor into its cells, or, once in the cells, to process the pro-inhibitor into active inhibitor.

The pro-inhibitor is preferably present in the growth medium at a concentration in the range 0.5 to 20 mg l⁻¹, preferably at least 0.75 mg l⁻¹ and no more than 10 mg l⁻¹, for instance about 1 mg l⁻¹.

The composition of the invention contains a complementary detection system to allow differentiation between the target Salmonella species and any other surviving commensal bacteria. The complementary detection system is a chromogenic enzyme substrate indicator which causes target bacterial colonies to appear visually distinct from commensal bacterial colonies. The medium may contain two or more such substrates. In this specification the term chromogenic includes fluorigenic.

The composition used to produce the selective medium preferably comprises sufficient nutrients to support bacterial growth and substances that act as growth factors (growth promoters) for the target Salmonella species. The composition which is used to produce the selective medium may produce a solid or a liquid selective medium, usually upon addition of water alone, or alternatively water and also support compound such as agar, and/or additional nutrients, growth promoters, growth inhibitors etc.

The pro-inhibitor i.e. the suicide substrate, of the selective medium is an oligo-peptidyl or amino acyl derivative of an antibacterial molecule. The selective medium should preferably be free from peptides which could antagonise the use of such peptide or amino acyl derivatives of antibacterial agents, for instance by competing with transport or hydrolytic systems on or in converting target species.

The pro-inhibitor is an amino-acyl or peptidyl compound, for instance which is converted to the inhibitor by hydrolysis of the amino acyl or peptidyl moiety, as the case may be. The pro-inhibitor is an N-aminoacyl or N-peptidyl derivative of an α-amino alkyl phosphonic acid, as represented by the general formula I.

As used in this specification, the term "lower alkyl" means a straight chain or branched-chain alkyl group which preferably contains from 1 to 6 carbon atoms (e.g. methyl, ethyl, propyl, isobutyl and the like). The term "aryl" preferably comprises mono-nuclear groups such as phenyl, which may be substituted in one or more positions with hydroxy, halogen, nitro, lower alkyl or lower alkoxy substituents. The term "halogen" means fluorine, chlorine, bromine and iodine and the term "lower alkoxy" means groups of the structure -O-(lower alkyl) wherein the lower alkyl group is as defined earlier. The expression "the characterising group of an alpha-amino acid of the type normally found in proteins" is used to mean the residue R in a natural alpha-amino acid of the general formula 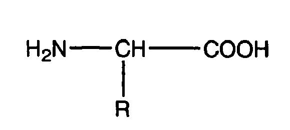 which is of the type normally occurring in proteins. Thus, for example, if the amino-acid is alanine, then the residue R represents the methyl group, in leucine the residue R represents the isobutyl group and in glutamic acid the residue R represents the 2-carboxyethyl group. R can also represent a residue which is linked with the amino nitrogen (with the loss of one of the hydrogen atoms attached thereto), thus forming a nitrogen-containing ring such as in proline and pyroglutamicacid. The term "lower cycloalkyl" means cyclic hydrocarbon groups containing 3 to 6 carbon atoms, such as cyclopropyl, cyclobutyl, cyclopentyl, cyclohexyl.

It will be appreciated that when n in formula I stands for 2 or 3, the value of R² can be the same or different.

Preferred compounds of formula I hereinbefore are those wherein R² and R³ each is hydrogen, methyl, isopropyl, isobutyl, benzyl, y-aminobutyl or 2-pyrrolidinyl, R¹ is hydrogen or methyl, R⁴ is hydroxy and n is zero or 1.

Examples of compounds of formula I hereinbefore are:
(L-alanylamino)-methylphosphonic acid
(L-valylamino)-methylphosphonic acid
(L-leucylamino)-methylphosphonic acid
(L-lysylamino)-methylphosphonic acid
L-phenylalanylamino)-methylphosphonic acid
(1R)-1-(L-alanylamino)-ethylphosphonic acid
(1R)-1-glycylamino-ethylphosphonic acid
(1R)-1-(L-alanylamino)-benzylphosphonic acid
(1R)-1-(L-lysylamino)-ethylphosphonic acid
(1R)-1-(L-leucylamino)-ethylphosphonic acid
(1R)-1-(L-alanylamino)-2-phenyl-ethylphosphonic acid
(1R)-1-(L-phenylalanylamino)-ethylphosphonic acid
(1R)-1-(L-valylamino)-ethylphosphonic acid
(L-alanyl-L-alanylamino)-methylphosphonic acid
(L-leucyl-L-alanylamino)-methylphosphonic acid
(L-alanyl-L-leucylamino)-methylphosphonic acid
(L-alanyl-L-phenylalanylamino)-methylphosphonic acid
(L-phenylalanyl-L-phenylalanylamino)-methylphosphonic acid
(L-phenylalanyl-L-alanylamino)-methylphosphonic acid
(1R)-1-(glycyl-L-alanylamino)-ethylphosphonic acid
(1R)-1-(L-valyl-L-alanylamino)-ethylphosphonic acid
(1R)-1-(L-phenylalanyl-L-alanylamino)-ethylphosphonic acid
(1R)-1-(L-prolyl-L-alanylamino)-ethylphosphonic acid
(L-alanyl-L-alanyl-L-alanylamino)-methylphosphonic acid
(1R)-1-(L-alanyl-L-alanyl-L-alanylamino)-ethylphosphonic acid
(1R)-1-(glycyl-L-alanyl-L-alanylamino)-ethylphosphonic acid
(1R)-1-(L-prolyl-L-alanyl-L-alanylamino)-ethylphosphonic acid
(1R)-1-(glycyl-glycyl-L-alanylamino)-ethylphosphonic acid
(1R)-1-(L-alanyl-L-alanyl-L-alanyl-L-alanylamino)-ethylphosphonic acid and
[(L-alanylamino)methyl]-methylphosphonic acid.

The α-amino phosphonic acids may be synthesised as described in US-A-4331591, US-A-4016148 and Atherton *et al* (op. cit.). Alafosfalin is commercially available.

A particularly preferred embodiment of the selective medium described above uses an amino acyl derivative of fosfalin (1-aminoethylphosphonic acid) as the pro-inhibitor. The preferred peptidyl or amino acyl derivative of fosfalin is alafosfalin (L-alanyl-1-aminoethylphosphonic acid). Alafosfalin, the preferred suicide substrate, may be used in selective media for screening bacterial samples since the target bacteria are Salmonella species.

In a selective medium suitable for target Salmonella, preferably included are growth factors for Salmonella species (which may be inhibitors of other Gram-positive species) for example sodium deoxycholate and sodium citrate.

The selective media also comprises one or more chromogenic enzyme substrate indicator. Chromogenic substrates are known and may be selected by the skilled person based upon the target species, commensal species, the type of medium and available detection systems. One of the substrates may be based on β-galactosidase activity which commensal bacteria (such as *E. coli*) hydrolyse to generate a strongly coloured product whereas Salmonella species produce colonies of a lighter colour due to a secondary biochemical reaction.

A preferred embodiment of the suicide substrate for incorporation into culture medium is a known bacterial substrate its chemical name is L-alanyl-1-aminoethylphosphonic acid and is also known as alafosfalin. This substrate, and a range of related compounds, has, in the inventor's belief, been proposed purely for therapeutic purposes and has been tested in MIC tests. Alafosfalin is taken up by bacteria at different rates via a stereospecific permease and may be cleaved intracellularly by a bacterial aminopeptidase to yield fosfalin (1-aminoethylphosphonic acid). This released compound interferes with bacterial metabolism via interaction with the alanine racemase enzyme responsible for generating D-alanine. This interaction may result in growth inhibition. The rate of uptake of the substrate, the rate of hydrolysis and the sensitivity of the racemase enzyme are all factors which will determine the degree of bacterial inhibition by the substrate. Consequently, different bacteria are inhibited to different degrees by this and related substrates as described in a series of papers produced by workers at Roche cited in Atherton *et al., op. cit..* Alafosfalin or analogues of the general formula I generally inhibit Gram-negative commensals.

The inhibitory activity of alafosfalin is antagonised by the presence of small peptides and the substrate is therefore inactive in normal bacteriological media which are based on peptones. The composition and medium of the present invention is preferably substantially free of other small peptides.

The following examples illustrate the invention.

### Example 1 - Screen

In order to investigate the usefulness of alafosfalin for isolation of Salmonella we performed determinations of the minimum inhibitory concentration (MIC) of alafosfalin for 433 Gram negative aerobic bacterial strains. This was performed using procedures described by Atherton *et al (op. cit.).* It was found that the mean minimum inhibitory concentration (MIC) for 61 strains of non-typhi Salmonella was 10.2 mg/l and all strains showed a MIC above 1 mg/l. In contrast the mean MIC of alafosfalin against 108 strains of *E.coli* (by far the commonest faecal commensal) was 0.7 mg/l. In addition, most other commensal bacteria showed a lower mean MIC than Salmonella spp. These finding suggested that alafosfalin was an excellent candidate as a selective agent for the isolation of Salmonella from faeces.

### Example 2 - Medium

We designed a modified medium for the isolation of Salmonella which allowed for the action of alafosfalin i.e. it was free from peptide antagonists. The composition is as follows:-

| | g |
|---|---|
| L-Arginine | 0.1 |
| L-Aspartic acid | 0.1 |
| L-Cysteine | 0.005 |
| Glycine | 0.1 |
| L-Histidine | 0.1 |
| L-Isoleucine | 0.1 |
| L-Leucine | 0.1 |
| L-Lysine-HCl | 0.1 |
| L-Methionine | 0.005 |
| L-Phenylalanine | 0.1 |
| L-Proline | 0.1 |
| L-Serine | 0.1 |
| L-Threonine | 0.1 |
| L-Tryptophan | 0.1 |
| L-Tyrosine | 0.1 |
| L-Valine | 0.1 |
| | |
| Guanine | 0.01 |
| Uracil | 0.01 |
| Cytosine | 0.01 |
| Adenine | 0.01 |
| | |
| Sodium Citrate | 6.5 |
| Magnesium sulphate | 0.1 |
| Ammonium sulphate | 1 |
| Yeast Extract | 0.1 |
| Dipotassium hydrogen phosphate | 7 |
| Potassium dihydrogen phosphate | 2 |
| | |
| Ferric ammonium citrate | 0.5 |
| 5-bromo-4-chloro-3-indolyl-alpha-D-galactoside | 0.08 |
| | |
| 3,4-cyclohexenoesculetin-β-D-galactoside | 0.3 |
| isopropyl-thio-β-D-galactoside | 0.03 |
| | |
| Bacteriological agar | 10 |
| De-ionised water | 1000 ml |

The above mixture is sterilised at 116 °C for 10 minutes cooled to below 100 C and to the mixture 0.001 g alafosfalin and 0.5 g sodium deoxycholate added. After preparation, this medium was poured into petri dishes to prepare plates.

XLD and ABC medium were purchased from Lab M, Bury UK and Hektoen Enteric agar and SM-ID were purchased from bioMerieux, Basingstoke UK. CHROMagar Salmonella and Rambach agar were obtained from M-tech Diagnostics, Warrington, UK. These were all prepared in exact accordance with manufacturer's instructions.

The 433 strains were inoculated onto each of the media as described above and the number of each species growing on each agar was calculated as a percentage of the total number of strains of each species that were tested.

**Table 1: Percent Recovery of various faecal bacteria on various Salmonella selective agars**

| | No. tested | ABC | ABC* | CS | HEK | RAM | SM-ID | XLD |
|---|---|---|---|---|---|---|---|---|
| Salmonella spp. | 61 | 100 | 100 | 100 | 100 | 100 | 100 | 100 |
| Total non-Salmonellae | 372 | 87.9 | 54.6 | 77.4 | 70.4 | 79.8 | 96.5 | 90.9 |
| Total E.coli | 108 | 96.3 | 10.2 | 95.4 | 45.4 | 98.1 | 100 | 78.7 |

| | | | | | | | | |
|---|---|---|---|---|---|---|---|---|
| ABC* refers to modified ABC medium containing 1 mg/l alafosfalin. | | | | | | | | |

- CS -: CHROMagar Salmonella
- HEK -: Hekloen agar
- XLD -: Xylose lysine decarboxylase
- RAM -: Rambach
- SM-ID -: trade mark

All of the Salmonella strains tested grew well on all of the agars including the novel medium incorporating 1 mg/l alafosfalin. It was found however that this new medium was superior in the inhibition of non-Salmonella strains in that 45.4% of strains showed no sign of growth on this medium. The next best agar was Hektoen enteric agar which inhibited 29.6% of non-Salmonella strains. Of greater significance was the fact that alafosfalin was especially selective against *E.coli -* by far the commonest aerobic faecal commensal. 89.8% of *E.coli* did not grow on this medium and this was far superior to any of the commercially available agars that were tested. In particular, the chromogenic agars that were tested were particularly poor at inhibiting *E.coli.* It was also note worthy that the colours generated by bacteria growing on the new medium were the same as the colours produced by the same strains on ABC medium. This showed that the alafosfalin did not interfere with the operation of the chromogenic substrates provided of course that bacteria were allowed to grow.

This example has shown that inclusion of a suicide substrate, in this case alafosfalin, offers a substantial improvement for the selective isolation of Salmonella when compared to the best existing technologies in the field of chromogenic media.

### Example 3

Faecal samples routinely sent to the Freeman Hospital Microbiology Department and the Regional Public Health Laboratory, Newcastle Upon Tyne were inoculated onto ABC, the modified ABC and the Hektoen agars used in Example 2, using the following method. Approximately 1 g of solid faeces or 1 ml of liquid faeces were emulsified in 10 ml of sterile saline (0.75%). 10 µl of this suspension was then inoculated onto each of the three media and streaked to obtain single colonies of bacterial growth. 9 ml of each faecal suspension was inoculated into 9 ml of double-strength selenite broth for enrichment. All plates and broths were incubated at 37°C for 24 hours. Plates were examined for the presence of Salmonella and all suspect colonies were investigated using standard biochemical and serological tests. Broths were subcultured onto the three media (10 µl of broth per plate as described above). These plates were incubated for 24 hours and analysed for suspect colonies as described.

A total of 797 faecal samples were processed. From these samples a total 33 Salmonella strains were recovered via enrichment through selenite broth. All 33 strains were recovered on all of the media after enrichment through selenite broth. The enrichment process required a total of 48 hours for isolation of Salmonella. The results achieved by the different media after direct inoculation of faeces ("direct culture") showed differences between the three media. These differences are summarised in Table 2.

**Table 2: Recovery of Salmonella species on various agars following direct inoculation**

| | **ABC Medium** | **Modified ABC Medium** | **Hektoen-Enteric agar** |
|---|---|---|---|
| No. of Salmonella Recovered | 17 | 25 | 18 |
| % Recovery | 51.5 | 75.8 | 54.6 |
| % Positivity | 2.1 | 3.1 | 2.3 |
| No. False Positives | 6 | 4 | 83 |
| % False Positivity | 0.75 | 0.5 | 10.41 |

It is clear from the table that modified ABC medium containing alafosfalin allowed for a significantly greater recovery of Salmonella after direct culture than either of the two reference media used. This was attributable to a substantial reduction in the normal flora which was allowed to grow on the medium containing alafosfalin. It is also apparent that the modified ABC medium was no less specific for Salmonella than ABC medium and it was substantially better than Hektoen-Enteric medium. These results prove that inclusion of the suicide substrate alafosfalin significantly enhances the recovery of Salmonella following direct culture. Although all Salmonella strains were recovered after enrichment, irrespective of the medium used, these results were delayed by the 24 hours required for the enrichment process.

## Claims

1. A method of culturing bacteria and detecting a target species of bacteria in which a sample containing a mixture of several bacteria suspected of containing target species of bacteria which is a salmonella species is incubated in the presence of a growth medium comprising a growth affecting compound, **characterised in that** the growth affecting compound is a pro-inhibitor which is non-toxic to target bacteria but which is convertable intracellularly within at least some bacterial species including at least one bacterial species present in the sample, the converting microbe, to form an inhibitor which kills or prevents division of the converting microbe, and which is an aminoacyl or oligopeptidyl derivative of an inhibitor;
in which the pro-inhibitor is represented by the general formula I 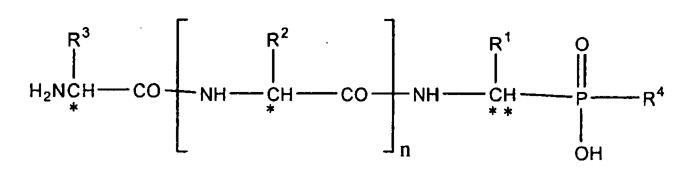 wherein R¹ is hydrogen, C₁-C₆ alkyl, C₁-C₆ cycloalkyl, (C₁-C₆ cycloalkyl)-(C₁-C₆ alkyl), aryl or aryl-(C₁-C₆ alkyl) said substituents other than hydrogen being optionally substituted by one or more of amino, hydroxy, thio, methylthio, carboxy or guanidino so as to form the characterising group of a naturally occurring L alpha-amino acid; R² and R³ each represent the characterising group of an alpha-amino acid of the type normally found in proteins with the proviso that R³ cannot be hydrogen when n is zero and R¹ is hydrogen or phenyl; R⁴ is hydroxy or methyl; n is zero, 1, 2 or 3; the single asterisks denote that the configuration at the carbon atom so-marked is L; and the double asterisk denotes that, when R¹ is other than hydrogen, the configuration at the carbon atom so-marked is that which would be obtained by replacing the carboxyl group of a naturally occurring L alpha-amino acid by a phosphorus moiety, and their pharmaceutically acceptable salts;
wherein the growth medium comprises at least one chromogenic substrate which is enzymically convertable to a differently coloured or fluorescing compound by the target microbe.

2. A method according to claim 1, in which the pro-inhibitor is intracellularly cleavable.

3. A method according to claim 2, in which the pro-inhibitor is hydrolytically cleavable.

4. A method according to claim 3, in which the cleavage is enzymatic.

5. A method according to claim 1, in which the pro-inhibitor is an aminoacyl or oligopeptidyl derivative of fosfalin.

6. A method according to claim 5, in which the pro-inhibitor is alafosfalin.

7. A method according to claim 1, in which the growth medium comprises growth factors for the target salmonella species, and/or selective compounds.

8. A method according to claim 7, comprising sodium deoxycholate and/or sodium citrate.

9. A method according to any preceding claim, in which the said converting microbe is *E. coli.*

10. A method according to any preceding claim, in which the growth medium is a solid medium.

11. A method according to claim 10, in which the growth medium is an agar medium.

12. A composition for making up into a bacterial growth medium comprising nutrients capable of supporting growth of a sample of mixed enteric bacteria and a pro-inhibitor which is non-toxic to bacteria of interest which are salmonella species but which is convertable intracellularly within at least some bacterial species of interest, the converting microbe, to form an inhibitor which kills or prevents division of the converting microbe, and which is an aminoacyl or peptidyl derivative of an inhibitor;
in which the pro-inhibitor is represented by the general formula I 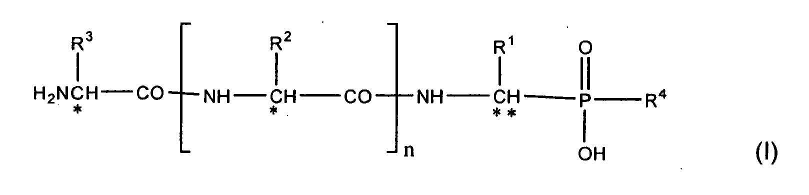 wherein R¹ is hydrogen, C₁-C6 alkyl, C₁-C₆ cycloalkyl, (C₁-C₆ cycloalkyl)-(C₁-C₆ alkyl), aryl or aryl-(C₁-C₆ alkyl) said substituents other than hydrogen being optionally substituted by one or more of amino, hydroxy, thio, methylthio, carboxy or guanidino so as to form the characterising group of a naturally occurring L alpha-amino acid; R² and R³ each represent the characterising group of an alpha-amino acid of the type normally found in proteins with the proviso that R³ cannot be hydrogen when n is zero and R¹ is hydrogen or phenyl; R⁴ is hydroxy or methyl; n is zero, 1, 2 or 3; the single asterisks denote that the configuration at the carbon atom so-marked is L; and the double asterisk denotes that, when R¹ is other than hydrogen, the configuration at the carbon atom so-marked is that which would be obtained by replacing the carboxyl group of a naturally occurring L alpha-amino acid by a phosphorus moiety, and their pharmaceutically acceptable salts;
further comprising at least one chromogenic substrate which is enzymically convertable to a differently coloured or fluorescing compound by the target salmonella species.

13. A composition according to claim 12, in which the pro-inhibitor is intracellularly cleavable.

14. A composition according to claim 13, in which the pro-inhibitor is hydrolytically cleavable.

15. A composition according to claim 13, in which the cleavage is enzymatic.

16. A composition according to claim 12, in which the pro-inhibitor is an aminoacyl or oligopeptidyl derivative of fosfalin.

17. A composition according to claim 16, in which the pro-inhibitor is alafosfalin.

18. A composition according to any of claims 12 to 17, which comprises growth factors for the target salmonella species, and/or selective compounds.

19. A composition according to claim 18, comprising sodium deoxycholate and/or sodium citrate.

20. A composition according to any of claims 12 to 19, which comprises a water-swellable compound.

21. A composition according to claim 20, in which the water-swellable compound is agar.

## Patentansprüche

1. Verfahren zur Kultivierung von Bakterien und zum Nachweis einer Bakterien-Zielspezies, in dem eine Probe, enthaltend ein Gemisch aus mehreren Bakterien, das vermutlich die Bakterien-Zielspezies enthält, bei der es sich um eine Salmonella-Spezies handelt, in Anwesenheit eines Wachstumsmediums inkubiert wird, umfassend eine auf das Wachstum einwirkende Verbindung, **dadurch gekennzeichnet, dass** die auf das Wachstum einwirkende Verbindung ein für Zielbakterien nicht toxischer Proinhibitor ist, der aber intrazellulär in mindestens einigen Bakterien-Spezies, einschließlich in mindestens einer in der Probe anwesenden Bakterien-Spezies, der umwandelnden Mikrobe, zur Bildung eines die umwandelnde Mikrobe abtötenden oder ihre Teilung verhindernden Inhibitors umwandelbar ist, und bei dem es sich um ein Aminoacyl- oder Oligopeptidyl-Derivat eines Inhibitors handelt;
worin der Proinhibitor durch die allgemeine Formel I dargestellt ist 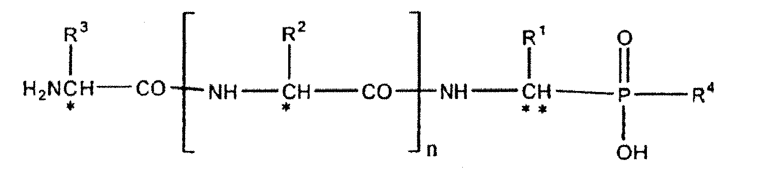 worin R¹ Wasserstoff, C₁-C₆-Alkyl, C₁-C₆-Cycloalkyl, (C₁-C₆-Cycloalkyl)-(C₁-C₆-alkyl), Aryl oder Aryl-(C₁-C₆-alkyl) ist, wobei andere Substituenten als Wasserstoff gegebenenfalls durch ein oder mehr von Amino, Hydroxy, Thio, Methylthio, Carboxy oder Guanidino zur Bildung der charakterisierenden Gruppe einer natürlich vorkommenden L-α-Aminosäure substituiert sind; R² und R³ jeweils die charakterisierende Gruppe einer α-Aminosäure des Typs darstellen, der in der Regel in Proteinen gefunden wird, mit der Maßgabe, dass R³ nicht Wasserstoff sein kann, wenn n 0 ist und R¹ Wasserstoff oder Phenyl ist; R⁴ Hydroxy oder Methyl ist; n 0, 1, 2 oder 3 ist; die einzelnen Sternchen bedeuten, dass die Konfiguration am so markierten Kohlenstoffatom L ist; und das Doppelsternchen bedeutet, dass wenn R¹ anders als Wasserstoff ist, die Konfiguration am so markierten Kohlenstoffatom die ist, die durch Austauschen der Carboxylgruppe einer natürlich vorkommenden L-α-Aminosäure durch einen Phosphorteil erhalten würde, und ihre pharmazeutisch verträglichen Salze;
worin das Wachstumsmedium mindestens ein chromogenes Substrat umfasst, das durch die Zielmikrobe enzymatisch in eine anders gefärbte oder fluoreszierende Verbindung umwandelbar ist.

2. Verfahren nach Anspruch 1, worin der Proinhibitor intrazellulär spaltbar ist.

3. Verfahren nach Anspruch 2, worin der Proinhibitor hydrolytisch spaltbar ist.

4. Verfahren nach Anspruch 3, worin die Spaltung enzymatisch ist.

5. Verfahren nach Anspruch 1, worin der Proinhibitor ein Aminoacyl- oder Oligopeptidyl-Derivat von Fosfalin ist.

6. Verfahren nach Anspruch 5, worin der Proinhibitor Alafosfalin ist.

7. Verfahren nach Anspruch 1, worin das Wachstumsmedium Wachstumsfaktoren für die Salmonella-Zielspezies und/oder selektive Verbindungen umfasst.

8. Verfahren nach Anspruch 7, umfassend Natriumdesoxycholat und/oder Natriumcitrat.

9. Verfahren nach einem der vorangehenden Ansprüche, worin die umwandelnde Mikrobe *E. coli* ist.

10. Verfahren nach einem der vorangehenden Ansprüche, worin das Wachstumsmedium ein festes Medium ist.

11. Verfahren nach Anspruch 10, worin das Wachstumsmedium ein Agarmedium ist.

12. Zusammensetzung zur Herstellung eines Bakterienwachstumsmediums, umfassend Nährstoffe, die zum Unterstützen des Wachstums einer Probe aus gemischten enterischen Bakterien fähig sind, und einen für die Bakterien von Interesse, bei denen es sich um Salmonella-Spezies handelt, nicht toxischen Proinhibitor, der aber intrazellulär in mindestens einigen Bakterienspezies von Interesse, der umwandelnden Mikrobe, zur Bildung eines die umwandelnde Mikrobe abtötenden oder ihre Teilung verhindernden Inhibitors umwandelbar ist, und bei dem es sich um ein Aminoacyl- oder Peptidyl-Derivat eines Inhibitors handelt;
worin der Proinhibitor durch die allgemeine Formel I dargestellt ist 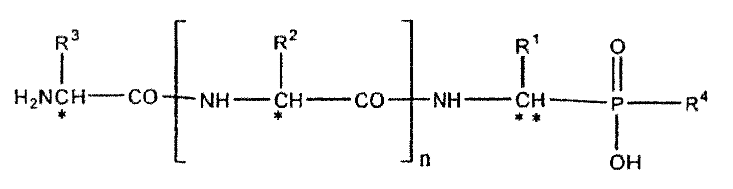 worin R¹ Wasserstoff, C₁-C₆-Alkyl, C₁-C₆-Cycloalkyl, (C₁-C₆-Cycloalkyl)-(C₁-C₆-alkyl), Aryl oder Aryl-(C₁-C₆-alkyl) ist, wobei andere Substituenten als Wasserstoff gegebenenfalls durch ein oder mehr von Amino, Hydroxy, Thio, Methylthio, Carboxy oder Guanidino zur Bildung der charakterisierenden Gruppe einer natürlich vorkommenden L-α-Aminosäure substituiert sind; R² und R³ jeweils die charakterisierende Gruppe einer α-Aminosäure des Typs darstellen, der in der Regel in Proteinen gefunden wird, mit der Maßgabe, dass R³ nicht Wasserstoff sein kann, wenn n 0 ist und R¹ Wasserstoff oder Phenyl ist; R⁴ Hydroxy oder Methyl ist; n 0, 1, 2 oder 3 ist; die einzelnen Sternchen bedeuten, dass die Konfiguration am so markierten Kohlenstoffatom L ist; und das Doppelsternchen bedeutet, dass wenn R¹ anders als Wasserstoff ist, die Konfiguration am so markierten Kohlenstoffatom die ist, die durch Austauschen der Carboxylgruppe einer natürlich vorkommenden L-α-Aminosäure durch einen Phosphorteil erhalten würde, und ihre pharmazeutisch verträglichen Salze;
ferner umfassend mindestens ein chromogenes Substrat, das durch die Salmonella-Zielspezies enzymatisch in eine anders gefärbte oder fluoreszierende Verbindung umwandelbar ist.

13. Zusammensetzung nach Anspruch 12, worin der Proinhibitor intrazellulär spaltbar ist.

14. Zusammensetzung nach Anspruch 13, worin der Proinhibitor hydrolytisch spaltbar ist.

15. Zusammensetzung nach Anspruch 13, worin die Spaltung enzymatisch ist.

16. Zusammensetzung nach Anspruch 12, worin der Proinhibitor ein Aminoacyl- oder Oligopeptidyl-Derivat von Fosfalin ist.

17. Zusammensetzung nach Anspruch 16, worin der Proinhibitor Alafosfalin ist.

18. Zusammensetzung nach einem der Ansprüche 12 bis 17, die Wachstumsfaktoren für die Salmonella-Zielspezies und/oder selektive Verbindungen umfasst.

19. Zusammensetzung nach Anspruch 18, die Natriumdesoxycholat und/oder Natriumcitrat umfasst.

20. Zusammensetzung nach einem der Ansprüche 12 bis 19, die eine in Wasser quellbare Verbindung umfasst.

21. Zusammensetzung nach Anspruch 20, worin die in Wasser quellbare Verbindung Agar ist.

## Revendications

1. Procédé de culture de bactéries et de détection d'une espèce cible de bactéries dans lequel un échantillon contenant un mélange de plusieurs bactéries soupçonnées de contenir une espèce de bactéries, laquelle est une espèce de salmonelle, est incubé en présence d'un milieu de croissance comprenant un composé affectant la croissance, **caractérisé en ce que** le composant affectant la croissance est un pro-inhibiteur qui est non toxique pour cibler les bactéries mais qui est convertible intracellulairement au sein d'au moins certaines espèces bactériennes comprenant au moins une espèce bactérienne présente dans l'échantillon, le microbe de conversion, pour former un inhibiteur qui tue ou empêche la division du microbe de conversion, et qui est un dérivé aminoacyle ou oligopeptidyle d'un inhibiteur ;
dans lequel le pro-inhibiteur est représenté par la formule générale I 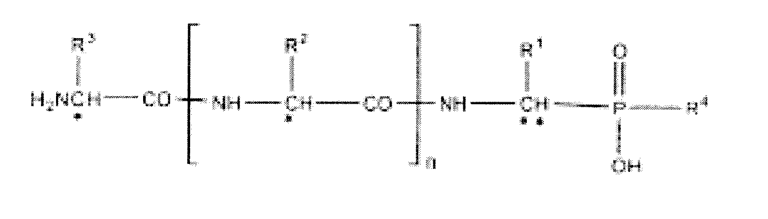 dans lequel R¹ est hydrogène, C₁-C₆-alkyle, C₁-C₆ cycloalkyle, (C₁-C₆ cycloalkyle)-(C₁-C₆ alkyle), aryle ou aryle-(C₁-C₆ alkyle), lesdits substituants autres que l'hydrogène étant optionnellement substitués par un ou plusieurs parmi les groupes amino, hydroxy, thio, méthylthio, carboxy ou guanidino de manière à former le groupe qui caractérise un acide L alpha-aminé présent à l'état naturel ; R² et R³ représentent chacun le groupe qui caractérise un acide alpha-aminé du type trouvé normalement dans les protéines à condition que R³ ne soit pas hydrogène quand n est zéro et R¹ est hydrogène ou phényle ; R⁴ est hydroxy ou méthyle ; n est zéro, 1, 2 ou 3 ; les astérisques uniques indiquent que la configuration au niveau de l'atome de carbone ainsi repéré est L ; et le double astérisque indique que, quand R¹ est autre que hydrogène, la configuration au niveau de l'atome carbone ainsi repéré est celle qui serait obtenue par remplacement du groupe carboxyle d'un acide L-alpha aminé présent à l'état naturel par une fraction phosphore, et leurs sels de qualité pharmaceutique ;
dans lequel le milieu de croissance comprend au moins un substrat chromogène qui est enzymatiquement convertible en un composé coloré différemment ou fluorescent par le microbe cible.

2. Procédé selon la revendication 1, dans lequel le pro-inhibiteur est intracellulairement clivable.

3. Procédé selon la revendication 2, dans lequel le pro-inhibiteur est hydrolytiquement clivable.

4. Procédé selon la revendication 3, dans lequel le clivage est enzymatique.

5. Procédé selon la revendication 1, dans lequel le pro-inhibiteur est un dérivé aminoacyle ou oligopeptidyle de fosfaline.

6. Procédé selon la revendication 5, dans lequel le pro-inhibiteur est l'alafosfaline.

7. Procédé selon la revendication 1, dans lequel le milieu de croissance comprend des facteurs de croissance pour l'espèce de salmonelle cible et/ou des composés sélectifs.

8. Procédé selon la revendication 7, comprenant du désoxycholate de sodium et/ou du citrate de sodium.

9. Procédé selon une quelconque revendication précédente, dans lequel ledit microbe de conversion est l'*E*. *Coli.*

10. Procédé selon une quelconque revendication précédente, dans lequel le milieu de croissance est un milieu solide.

11. Procédé selon la revendication 10, dans lequel le milieu de croissance est un milieu d'agar.

12. Composition pour réaliser un milieu de croissance bactérienne comprenant des nutriments capables de soutenir la croissance d'un échantillon de bactéries entériques mélangées et d'un pro-inhibiteur qui est non toxique pour les bactéries visées, lesquelles sont des espèces de salmonelle, mais qui est convertible intracellulairement au sein d'au moins une partie des espèces bactériennes visées, le microbe de conversion, pour former un inhibiteur qui tue ou empêche la division du microbe de conversion, et qui est un dérivé aminoacyle ou peptidyle d'un inhibiteur ;
dans lequel le pro-inhibiteur est représenté par la formule générale I 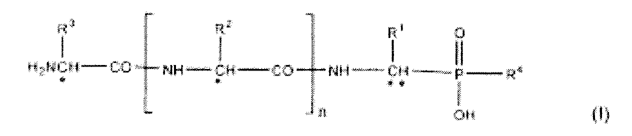 dans lequel R¹ est hydrogène, C₁-C₆-alkyle, C₁-C₆ cycloalkyle, (C₁-C₆ cycloalkyle)-(C₁-C₆ alkyle), aryle ou aryle-(C₁-C₆ alkyle), lesdits substituants autres que l'hydrogène étant optionnellement substitués par un ou plusieurs parmi les groupes amino, hydroxy, thio, méthylthio, carboxy ou guanidino de manière à former le groupe qui caractérise un acide L alpha-aminé présent à l'état naturel ; R² et R³ représentent chacun le groupe qui caractérise un acide alpha-aminé du type trouvé normalement dans les protéines à condition que R³ ne soit pas hydrogène quand n est zéro et R¹ est hydrogène ou phényle ; R⁴ est hydroxy ou méthyle ; n est zéro, 1, 2 ou 3 ; les astérisques uniques indiquent que la configuration au niveau de l'atome de carbone ainsi repéré est L ; et le double astérisque indique que, quand R¹ est autre que hydrogène, la configuration au niveau de l'atome carbone ainsi repéré est celle qui serait obtenue par remplacement du groupe carboxyle d'un acide L-alpha aminé présent à l'état naturel par une fraction phosphore, et leurs sels de qualité pharmaceutique ;
comprenant en outre au moins un substrat chromogène qui est enzymatiquement convertible en un composé coloré différemment ou fluorescent par les espèces de salmonelle cibles.

13. Composition selon la revendication 12, dans lequel le pro-inhibiteur est intracellulairement clivable.

14. Composition selon la revendication 13, dans lequel le pro-inhibiteur est hydrolytiquement clivable.

15. Composition selon la revendication 13, dans lequel le clivage est enzymatique.

16. Composition selon la revendication 12 dans lequel le pro-inhibiteur est un dérivé aminoacyle ou oligopeptidyle de fosfaline.

17. Composition selon la revendication 16 dans lequel le pro-inhibiteur est l'alafosfaline.

18. Composition selon l'une quelconque des revendications 12 à 17 qui comprend des facteurs de croissance pour l'espèce de salmonelle cible et/ou des composés sélectifs.

19. Composition selon la revendication 18 comprenant du désoxycholate de sodium et/ou du citrate de sodium.

20. Composition selon l'une quelconque des revendications 12 à 19 qui comprend un composé dilatable par l'eau.

21. Composition selon la revendication 20 dans lequel le composé dilatable par l'eau est l'agar.
